# EUROPEAN PATENT APPLICATION

(11) **EP 2 015 072 A1**
(43) Date of publication of application: **14.01.2009**
(21) Application number: 07112356.6
(22) Date of filing: 12.07.2007
(51) Int. Cl.: G01N 33/557, G01N 33/53

(54) **Ultrasound elution of biological ligands**

(71) Applicant: LEK Pharmaceuticals d.d., 1526 Ljubljana (SI)
(72) Inventor: Lunder, Mojca, 1000 Ljubljana (SI); Bratkovic, Tomaz, 1000 Ljubljana (SI); Urleb, Uros, 1000 Ljubljana (SI); Kreft, Samo, 1000 Ljubljana (SI); Strukelj, Borut, 1000 Ljubljana (SI); Ekar, Petra, 4248 Lesce (SI)
(74) Representative: Wichmann, Hendrik

(57) **Abstract**

The present invention discloses a method for analyzing the binding of a biological ligand to a target, comprising the steps of (1) providing one, two, or more different candidates for a biological ligand, (2) contacting said candidate(s) with a target, wherein for each candidate the target is the same or has the same structure, (3) applying ultrasound to the target-bound candidates for a ligand. In preferred embodiments, the invention relates to the use of the invention for screening biological libraries and in the context of phage display. Preferably, the ultrasound is applied at acidic pH. The ultrasound may be applied during an elution or a wash step. The candidate for a biological ligand is preferably chosen from the group of peptides, peptidomimetics, antibodies, nucleic acids, carbohydrates, saccharides, oligosaccharides, polysaccharides, and glycosylated peptides.

## Description

The present invention relates to analyzing biological ligands, particularly to detecting biological ligands having a higher or lower affinity than other biological ligands. The invention also relates to elution or washing methods in biological assays.

Specific interactions between biological molecules, e.g. between receptors and their respective ligands, play a key role in many biological processes. Therefore, identifying specific molecular interactions, in particular interactions between proteins or peptides, has attracted considerable interest and spawned an entire field of science known as proteomics. In turn, drug development is profiting from knowledge about such interactions and from modifying molecules in order to optimize binding according to desired characteristics.

In principle, candidates for ligands are brought into contact with an immobilized target. In a subsequent washing step, those candidates which do not bind are washed off. Alternatively, the ligands can be immobilized and the target molecules are washed off.

A typical example for such a method is phage display. Phage display is routinely used to select and optimize peptides or protein domains binding to given targets (Smith, G.P & Petrenko, V.A. Chem Rev. 97, 391-410 (1997); Szardenings, M. J. Recept. Signal Transduct. Res. 23, 307-49 (2003); Lowman, H.B. Annu. Rev. Biophys. Biomol. Struct. 26, 401-24 (1997). In principle, a collection of genes of interest (encoding proteins of interest) is introduced into bacteriophages. The DNA encoding the protein of interest is fused with a gene encoding one of the proteins that form the viral coat. When this bacteriophage infects E. coli, it replicates, producing phage particles that display the hybrid protein on the outside of their coats. These phages are contacted with an immobilized target. Phages displaying a protein binding the target will stick to the immobilized target, whereas unbound phages can be washed off from the target. The DNA from the bound phages can be isolated and thus a interaction partner can be identified.

However, it has turned out that the washing conditions to remove unbound or unspecifically bound candidates for ligands are difficult to determine and sometimes there is even a total lack of appropriate washing conditions. For example, if the washing conditions in phage display are too stringent, all phages will be eluted from the target. In turn, if the washing conditions are not stringent enough, many phages unspecifically binding to the target will remain bound (Lowman, H.B. Annu. Rev. Biophys. Biomol. Struct. 26, 401-24 (1997); Dennis, M.S., Lowman, H.B. Phage selection strategies for improved affinity and specificity of proteins and peptides. In Phage Display: A Practical Approach (eds. Clackson, T. & Lowman, H.B.) 61-83 (Oxford University Press, New York, USA, 2004)). Thus, it is typically necessary to amplify the bound phages and to stepwise enrich the binding phages by cycles of binding, washing and amplifying the bound phages.

Frequently, washing protocols have to be tediously adapted to a particular target, including the usage of different types and concentrations of surfactants, ionic strengths, different pH and even target protein or known ligands in washing buffer (Dennis, M.S., Lowman, H.B. Phage selection strategies for improved affinity and specificity of proteins and peptides. In Phage Display: A Practical Approach (eds. Clackson, T. & Lowman, H.B.) 61-83 (Oxford University Press, New York, USA, 2004); Yu, H.Q., Dong, X.Y. & Sun, Y. Biochem. Eng. J. 18, 169-175 (2004); Lunder, M., Bratkovi 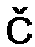 , T., Kreft, S. & 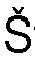 trukelj, B. J. Lipid. Res. 46, 1512-6 (2005); D'Mello, F. & Howard, C.R. J. Immunol. Method. 247, 191-203 (2001)).

The washing steps can be very tedious and time-consuming. Furthermore, e.g. surfactants and ionic strength of the washing buffer may also affect the folding of the proteins of interest and cause artificial results to be obtained. In phage display, it is possible (although tedious) to partially overcome the limitations of washing conditions by stepwise enriching the binding phages. In other methods such enrichment may not be possible at all (e.g. in Western blotting or screening of spotted protein or peptide libraries) and appropriate washing methods become even more crucial.

In summary, there is a need for an improved method for detecting and/or analyzing the binding of ligands to targets, in particular for distinguishing between binding with higher or lower affinity or specificity. In particular, there is a need for faster, simpler, more specific and more efficient methods.

These needs and further needs are addressed by the present invention.

In a first embodiment, the invention relates to a method for analyzing the binding of a biological ligand to a target, comprising the steps of
(1) providing one, two, or more different candidates for a biological ligand,
(2) contacting said candidates with a target, wherein for each candidate the target is the same or has the same structure,
(3) applying ultrasound to the target-bound candidates for a ligand.

The invention also relates to use of ultrasound in a method for analyzing the binding of a biological ligand to a target, particularly in the context of a method as defined above.

Previously, ultrasound has been known as a means for cleaning hard surfaces, e.g. for cleaning of laboratory equipment, jewellery or glasses. Ultrasound of approximately 3 to 15 Mhz is being applied in clinical sonographic methods. In biomicroscopy, frequencies of 20 to 55 MHz or even higher are being used, see e.g. the Mouse Imaging Center, Toronto, CA (http://www.mouseimaging.ca/research/ubm.html). Ultrasound of approximately 150 MHz has been suggested for the mixing of liquids in microarray applications. (see e.g. Rathgeber, A., Wassermeier, M., and Wixforth A (2005). Acoustic 'distributed source' mixing of smallest fluid volumes. Journal of ASTM International, vol. 2, issue 6).

However, ultrasound has also destructive properties to biological material and has been used as a means to disrupt cells or to fragment DNA (e.g. sonicated salmon sperm DNA is being used in several applications in molecular biology). Application of ultrasound of 10 to 40 kHz via a metal rod can be used to disrupt cells and organelles. High intensity focused ultrasound (HIFU) of a frequency from 250 to 2000 kHz has been used to target and destroy tumors (F Wu, Z-B Wang, Y-De Cao, W-Z Chen, J Bai, J-Z Zou and H Zhu. A randomised clinical trial of high-intensity focused ultrasound ablation for the treatment of patients with localised breast cancer. British Journal of Cancer (2003) 89, 2227-2233).

In the context of the invention it has been found that the application of ultrasound can improve the washing or elution steps in ligand-target interaction studies. It was quite unexpected that ultrasound could be applied without significant damage to the molecules concerned. Surprisingly, not only large proteins but even phages, i.e. large protein complexes containing DNA, have been found to remain sufficiently unimpaired to allow for further proliferation (see Examples).

Remarkably, it was found in the course of the invention that ultrasound can advantageously be applied during washing and/or particularly during elution. The inventors have realized that not only washing but also (or particularly) the step of elution can be decisive for the outcome. It has been found that ultrasound can serve to elute those ligands which are bound with higher affinity than can usually be eluted by means of different methods. Thus, the invention allows to improve efficiency of eluting the best ligands. One explanation for this effect may be that previous methods have failed to elute most or all ligands. Thus, previously particularly the ligands binding with the highest affinity (sometimes considered as the "best" ligands) may have remained bound and been ignored during further analysis. Particularly, in methods involving multiple rounds of selection the ligands binding with the highest affinity may have been underrepresented in the eluate, thus considerably impeding the selection process.

Consequently, as the method of the invention allows even high affinity ligands to be efficiently eluted, the stringency of the preceding wash steps may be increased without compromising the overall efficiency of the method.

According to the method of the invention, one or more candidate(s) for a ligand are brought into contact with a target. The term "candidate for a ligand" is further defined below. Typically, a candidate which is capable of binding to the target will form a complex with the target. In contrast, a candidate which is not capable of binding to the target will typically remain unbound, e.g. in solution. Preferably, the candidate(s) are contacted with the target under conditions allowing the binding of such candidates which would be considered biological ligands (see further below). Therefore, the method may comprise additional step (2a) of allowing candidate(s) for a ligand to bind to the target. The person skilled in the art is familiar with suitable conditions for binding. In a preferred example, the contacting will be carried out in an aqueous solution, e.g. an appropriate buffer. The binding of candidate(s) is to the target is not necessary. For example, sometimes none of the candidate(s) provided can be considered a biological ligand. Therefore, binding of such candidate(s) can not be expected. Subsequently to contacting, a wash step may be employed in order to remove unbound ligand and/or target. However, a wash step will not always be necessary, e.g. if unbound ligand and/or target will not interfere with subsequent analysis. E.g. the target-candidate complex may be analyzed or detected directly (e.g. by means of surface plasmon resonance). Once one or more candidate(s) have bound to a target, it may be desirable to select, separate or isolate the respective candidate(s). For example, separating and/or isolating may be desirable, if the respective candidate(s) shall be further analyzed or amplified (for example, in phage display it may be desirable to analyze and/or amplify the respective binding phages).

In the course of the invention it has been found that ultrasound may be applied advantageously at different stages of the method described above. Ultrasound may be applied during a wash step (facilitating the removal of unbound candidates or of candidates binding with low affinity) and/or during a step of eluting the candidates bound to the target. Particularly, it has been found in the course of the invention that applying ultrasound in order to elute the target-bound candidates can quite substantially improve the yield of candidates binding with high affinity. It appears that ultrasound will help to elute particularly those candidates which bind with such high affinity that they are difficult to elute by other means. However, it is frequently desirable to elute particularly the candidates which bind with the highest affinity, as those candidates may be of most interest. Based on the finding that ultrasound may improve elution of candidates binding with high affinity, it appears that ultrasound may also increase the specificity and selectivity of analysis. This assumption is supported by the fact that it was possible to separate and identify high affinity phage clones in a single step of selection (see Examples).

The term "washing" or "wash step" is known to the person skilled in the art. Preferably, the term relates to removing candidate(s) for biological ligands which are not bound with high affinity and/or specificity to a target. The terms high affinity and specificity are known to the person skilled in the art and are defined elsewhere in this specification. Wash steps are known from many biological methods. In the most simple case, a wash step may be carried out by adding a solution or buffer of the same composition as being used for binding of the candidate(s) and subsequently removing said solution, thereby removing excess candidate(s). However, in many cases the wash solution will be of different composition as compared to the solution used for binding, e.g. in order to achieve a higher stringency of washing. The wash step may be reiterated for several times in order to sufficiently remove any excess candidate(s).

The term "elution" is known to the person skilled in the art. Preferably, the term relates to removing a biological ligand which is bound with high affinity and/or specificity to a target.

Thus, the elution step will usually be preceded by a wash step (of removing the candidates not being bound or being bound unspecifically or with lower affinity). The terms "high affinity" and "specificity" are known to the person skilled in the art and are defined elsewhere in this specification.

The term "ligand" is known to the person skilled in the art. In the context of the invention, the term ligand shall preferably be understood as relating to any candidate(s) which is/are capable of binding to the target. More preferably, the term ligand shall be understood as relating to any candidate capable of binding to the target specifically and/or with high affinity. The terms "specifically" and "high affinity" are defined elsewhere in this specification. Preferably, the term "binding" in this context relates to binding under appropriate conditions, more preferably in aqueous solution, even more preferably in a suitable buffer or under physiological conditions.

In the method of the invention, the candidate or the target may be immobilized, e.g. to a solid or semi-solid support. Such immobilization will help to more easily separate target-bound candidate(s) from candidates or targets which are not bound. However, it is not necessary that the target or the ligand are immobilized. It is also possible to detect a target-bound candidate, if the target and ligand are not immobilized, e.g. due to the larger size of such complex detectable by means of size analysis, e.g. size-exclusion chromatography. Such method also allows to identify ligands which bind the target with higher affinity (i.e. remain bound) and ligands which bind with lower affinity (and can be detected in the eluate).

The "target" according to the invention can be a single molecule or part of a molecule or it can be a number of molecules or parts of molecules. E.g. the target can be a particular epitope.

The method may e.g. be used to analyze whether a biological ligand capable of binding to the target is present in a particular sample. However, the method will also have many further applications, for example it will allow to detect, select, separate, isolate, identify, or optimize or otherwise study the binding of biological ligand to a target.

Thus, it will be appreciated that the method may comprise additional steps as considered appropriate by the person skilled in the art or as suggested according to further embodiments below. Such embodiments and additional steps may of course be used independently or in combination.

In another embodiment, the method may additionally comprise the step (4) of detecting the candidate(s) remaining bound to the target or detecting the candidate(s) not bound to the target. In this embodiment, a candidate remaining bound to the target is considered a biological ligand binding to the target. Such embodiment is advantagous for many screening applications in which the candidate of interest (either the bound or the not bound candidate) is detected in order to perform additional selection rounds. Detecting a bound candidate provides a pointer towards which candidate should be considered as a biological ligand. It may also indicate which candidate(s) should be used for further selection, optionally preceded by mutation or derivatization.

In another embodiment, the method may additionally comprise the step (4) of identifying the candidate(s) remaining bound to the target or identifying the candidate(s) not bound to the target. The term "identifying" as used in this context relates to elucidating the nature of the respective bound or not bound candidate. For example the molecular structure may be analyzed. Identification may e.g. be carried out by any analytical means deemed appropriate, e.g. by means of other (secondary) ligands (e.g. antibodies) specifically binding to the first ligand or by other means e.g. mass spectrometry, or peptide analysis as known to the person skilled in the art.

In yet another embodiment, the method may additionally comprise the step (4) of isolating the candidate(s) remaining bound to the target or isolating the candidate(s) not bound to the target. Such isolation will allow to use the respective candidate in further procedures, such as further selection rounds, optionally preceded by proliferation, mutation or derivatization of the candidate.

In yet another embodiment, the method may additionally comprise the step (4) of selecting the candidate(s) remaining bound to the target or selecting the candidate(s) not bound to the target. For example, such selection will allow to select a ligand binding with lower affinity or higher affinity as compared to another ligand. Such selection may also be part of an affinity selection method in phage display or a SELEX (systematic evolution of ligands by exponential enrichment) method in nucleic acid aptamer development.

In yet another embodiment, the method may additionally comprise the step (4) of separating the candidate(s) remaining bound to the target or separating the candidate(s) not bound to the target. For example, such selection will allow to select a ligand binding with lower affinity or higher affinity as compared to another ligand. Such selection may also be part of an affinity selection method in phage display or a SELEX (systematic evolution of ligands by exponential enrichment) method in nucleic acid aptamer development.

In further analogous embodiments, the method may also be used for optimizing a biological ligand, e.g. by methods of mutation and selection, which are generally known to the person skilled in the art. For example, the binding of candidate(s) for biological ligand(s) to different targets may be used in order to optimize or detect a ligand which selectively or specifically binds to one target but not to another target (e.g. by means of counter-SELEX, see US Pat. No. 5,580,737 "High-affinity nucleic acid ligands that discriminate between theophylline and caffeine"). In this context, it is also referred to the definition of specific binding as given elsewhere in this specification.

Therefore, the term "analyzing" relates to any kind of studying the binding of a candidate for a biological ligand to a target, particularly to detecting, selecting, separating, isolating, identifying, or optimizing a biological ligand. The method may also be used in the context of analyzing inhibitors or activators of a given target as a first step in the process of analyzing an inhibitor or activator (in particular of a biological target) is to analyze, particularly to detect, the binding of a candidate for an inhibitor or activator to its target.

It should be understood also the method and the mentioned uses can be applied not only to a single type of ligand but also to groups of ligands, e.g. to detect, select, separate, isolate, identify, optimize, or otherwise analyze groups of ligands with similar affinity or specificity.

As already mentioned, the method can also be used in the context of screening and selection methods involving several rounds of screening and selection. Consequently, the steps (1) to (3) as well as any step (4) as mentioned above may be repeated. However, the method provides the advantage, that the number of selection or screening rounds can be reduced as compared to previous methods. In many cases it will be sufficient to perform only one round of analysis, selection or screening. In fact, it was found that it was possible to select high affinity clones in a single selection round in phage display, which is quite unique (see Examples). With previous methods, it would usually have been necessary to perform at least two or three additional selection rounds.

If ultrasound is applied to the target-bound candidate during a wash step, then the ultrasound may enhance the specificity and/or effectiveness of removing any non-specifically bound target or candidate. The mechanism by which the invention works may be explained by different factors. The ultrasound may serve to break low-affinity or non-specific interactions between target and candidate ligand. However, the ultrasound may also serve to break unspecific and undesired interactions between candidate or a target and a support, a surface of a reaction vessel (e.g. microtiter plate well). In any of these cases, ultrasound serves to favor specific or high affinity binding between ligand and target to any unspecific or low affinity binding. In contrast, low-affinity or low-specificity bound candidate or ligand is dissociated and can be removed. However, further but yet unknown effects may also be present.

If ultrasound is applied during a wash step, the frequency and power of the ultrasound being applied are preferably chosen in a manner that preferentially the candidates which bind to the target with high affinity or sufficient specificity remain bound. The terms "high affinity" and "specificity" or "specifically binding" in this context are known to the person skilled in the art and are further explained elsewhere in this specification. Preferably, power and frequency are chosen to be sufficient to dissociate unspecifically bound ligand or target. The appropriate power and frequency can be easily determined by the person skilled in the art, e.g. by means of a preliminary test series. For example, a good starting point for such test would be a frequency of between 5 and 80 kHz and a power lower than the power suggested for elution of the target-bound candidate (see further below), e.g. between 10 and 100 W.

Applying of ultrasound to the target-bound candidate in an elution step may enhance the effectiveness of elution. If elution is preceded by stringent washing, the specificity of the method is greatly enhanced. As the effectiveness of elution is increased by ultrasound, the preceding wash steps can be carried out with increased stringency without compromising the yield of ligands analyzed. The ultrasound may help to break interactions between target and ligand. However, the ultrasound may also serve to break interactions between candidate or target and a support, e.g. the surface of a reaction vessel (e.g. microtiter plate well). However, further but yet unknown effects may also be present.

Depending on the setup of the method (particularly depending on whether the candidate or the ligand is immobilized), either the ligand or the target may be eluted. However, it is conceivable that the ligand may be eluted together with the candidate (e.g. as a ligand-target complex).

If ultrasound is applied in an elution step, the frequency and power of the ultrasound being applied are preferably chosen in a manner that a high fraction, preferably substantially most or all, of the target-bound candidates are eluted. Most preferably, the frequency and power should be sufficient to elute also ligands which bind to the target with high affinity and/or specificity. Preferably, power and frequency are chosen not to destroy a given functionality of the ligand (e.g. infectivity in case of a bacteriophage). The appropriate power and frequency can be easily determined by the person skilled in the art. A good starting point is a frequency higher than 10 or 15 kHz, more particularly higher than 17 kHz, higher than 19 kHz, higher than 20 kHz, higher than 22 kHz, higher than 25 kHz, higher than 30kHz, most partiularly higher than 40 kHz. A suitable upper limit can be determined by the person skilled in the art by performing simple comparative studies with respect to the particular target or ligand investigated. However, satisfying results can be obtained if the frequency is not higher than 100 kHz, more preferably not higher than 80 kHz, more preferably not higher than 70 kHz, most preferably not higher than 60 kHz. The suggested values of upper and lower limit may be combined as desired, suitable frequency ranges would therefore be e.g. between 10 and 100kHz, between 15 and 80 kHz, between 15 kHz and 70 kHz, between 25 kHz and 60 kHz, or between 30 and 70 kHz.

The most suitable power can be determined by the person skilled in the art, e.g. by performing simple comparative studies with respect to the particular target or ligand investigated and with respect to the particular conditions and ultrasound system being used. For example, a suitable starting point for appropriate power would be in the range of 15 to 400 W, more preferably in the range of 150 to 250 W, in the case of a microtiter plate (e.g. with 24, 48, 96, or 384 wells and so forth), particularly if the ultrasound is applied by means of a typical sonication water bath for cleaning of laboratory equipment. If the candidate is a rather unstable molecule, e.g. RNA, a good starting point for appropriate power might be rather at the lower end of the suggested power range given above. However, if the aim is to analyze (particularly to find, detect or identify) a ligand of high stability, then it may even be advantageous to use rather high power. Such may be the case e.g. in the selection of RNA aptamers having high stability. If the method is applied in several rounds of selection, it may also be considered to increase the power during the later rounds of selection and thus to increase the stringency.

Frequency and power may be combined as considered appropriate, e.g. a good starting point would be to use approximately between 30 and 70 kHz (e.g. approximately 50 kHz) combined with a power of 15 to 400 W (e.g. approximately 300W) in the case of a microtiter plate (e.g. with 24, 48, 96, or 384 wells and so forth), particularly if the ultrasound is applied by means of a typical sonication water bath for cleaning of laboratory equipment.

More detailed guidance about the appropriate frequency and power is also given in the Examples.

The ultrasound may be applied by different means, e.g. via a liquid or aqueous medium such as a water bath or directly onto a support to which the target or the ligand have been immobilized. However, if several candidates for ligands are analyzed in parallel (such as in case of a microtiter plate or an microarray), the ultrasound frequency and power should be sufficiently uniform or homogenous for all analyses being performed (or target-bound candidates being investigated), e.g. the frequency and power should be similar in all wells of a microtiter plate in which an analysis is performed. Systems for applying ultrasound directly to microarrays or laboratory microsystems are known to the person skilled in the art and may be adapted to the purpose of the present invention (see e.g. Rathgeber, A., Wassermeier, M., and Wixforth A (2005). Acoustic 'distributed source' mixing of smallest fluid volumes. Journal of ASTM International, vol. 2, issue 6).

The term "binding" relates to any kind of chemical bonding between a biological ligand and a target. Particularly, the term binding relates to any kind of non-covalent chemical bond formation, for example to formation of ionic bonds, hydrophobic interactions, hydrogen bonds, van der Waals bonds, and any other non-covalent bonds known to the person skilled in the art, as well as to any combinations of such bonds. Typically, a biological ligand will interact with its target via several different bonds, thus resulting in overall binding of higher affinity than achieved by a single bond. Preferably, for a molecule to be considered a biological ligand in the sense of the invention, the molecule should bind to the target with a dissociation constant Kd of preferably equal or less than 10⁻⁴ M, more preferably equal or less than 10⁻⁵ M, more preferably equal or less than 10⁻⁶ M, more preferably equal or less than 10⁻⁷ M, more preferably equal or less than 10⁻⁸ M, more preferably equal or less than 10⁻⁹ M, most preferably equal or less than 10⁻¹⁰ M. For example, a quite good biological ligand may bind its target (particularly a target protein) with a Kd of of 10⁻⁸ M to 10⁻¹⁰ M. Typical peptides analyzed or generated by methods of phage display may bind their target with a Kd of 10⁻⁵ M to 10⁻⁷ M The particular desired affinity will largely depend on the purpose for which the biological ligand is to be used.

The term "candidate for a biological ligand" relates to any kind of organic molecule, but preferably to peptides and derivatives thereof, nucleic acids and derivatives thereof, carbohydrates and derivatives thereof. For example, the candidate for a biological ligand may be chosen from the group of peptides, peptidomimetics, antibodies, nucleic acids, carbohydrates, saccharides, oligosaccharides, polysaccharides, and glycosylated peptides. All of these terms are known to the person skilled in the art.

The term "peptide" should be understood in its broadest sense and includes oligopeptides, polypeptides and proteins. Oligopeptides are understood as peptides having a length of up to 10 amino acids, polypeptides are understood as peptides having a length of more than 10 amino acids. Preferably, the peptides according to the invention have a length or more than 2, more preferably of more than 3, 4, 5, or 7 amino acids. The term "peptidomimetic" is also known to the person skilled in the art. More particularly, the term peptidomimetic relates to a small protein-like chain similar to a peptide, wherein the peptide is modified e.g. to have an altered backbone or to incorporate non-natural amino acids. Peptidomimetics may be designed to have advantageous properties compared to peptides. For example, a peptidomimetic can have a modified degradation property such as increased stability under physiological conditions. All definitions and preferably sizes concerning peptides as given above should be applied mutatis mutandis, to peptidomimetics as well

The term "antibody" as used herein includes both polyclonal and monoclonal antibodies, as well as fragments thereof, such a scFv (single chain variable fragment), Fv, Fab and F (ab)2 that are capable of binding a target. An antibody can be understood as a special form of a peptide.

The term "nucleic acid" according to the present invention relates to any kind of natural or artificial nucleic acid such as deoxyribonucleic acid (DNA), ribonucleic acid (RNA), and to nucleic acids with backbone structures other than phosphodiesters, e.g. phosphothiates or phosphoramidates, peptide nucleic acids (PNA), morpholinos, locked nucleic acids (LNA), glycol nucleic acids (GNA), and threose nucleic acids (TNA).

The term "carbohydrate" is known to the person skilled in the art. According to the present invention, the term carbohydrate relates to any kind of aldehyde or ketone related to sugars, including simple derivates (e.g. amino sugars, carboxylic acids) and polymers (such as polysaccharides).

The terms "oligosaccharide" and "polysaccharide" are readily understood by the person skilled in the art. More particularly, the term oligosaccharide relates to any kind of oligomer, made up of saccharide monomers and having a length of up to 50 monomer subunits. An oligosaccharide may be branched or not branched and it may contain only type of monomer (homo-oligomer) or different types of monomers (hetero-oligomer). The term polysaccharide more particularly relates to any kind of polymer made of saccharide monomers and having a length of more than 50 monomer subunits. A polysaccharide may be branched or not branched and it may contain only type of monomer (homo-polymer) or different types of monomers (hetero-polymer).

Glycosylated peptides, particularly glycoproteins, are known to the person skilled in the art. Such molecules may be obtained by expression of a peptide as defined above in mammalian cell culture or by in vitro glycosylation. The coupling may occur e.g. via the nitrogen of asparagine side chains or via the oxygen of serine or threonine residues. The sugar residues may include any kind of natual or non-natural saccharide, oligosacharide, polysaccharide, or derivative thereof, e.g. N-acetylgalactosamine (GalNAc), N-acetylglucosamine (GlcNAc), galactose, mannose, sialic acid, or fucose.

Preferably, the candidate is not a "small molecule", but an entity of higher molecular weight, preferably a weight of more than 200 Da, more preferably more than 500 Da, more preferably more than 1000 Da, most preferably more than 3000 Da. In the case of a peptide or derivative thereof, the candidate has preferably a weight or length corresponding to at least 5, 10, 15, 30, 50, or 100 amino acids. In the case of a nucleic acid or derivative thereof, the candidate has preferably a weight or length corresponding to at least 10, 15, 20, 30, 50, or 100 nucleotides in length.

Preferably, the candidate for a biological ligand may comprise a peptide (as may be the case e.g. in phage display) or it may be chosen from the group of peptides. The target may be any kind of substance to which another substance (particularly a biological ligand) is capable of binding non-covalently but specifically. Thus, the target should preferably contain a suitable epitope. Preferably, the target is not a metal or a non-naturally occuring polymer, e.g. plastic.

The biological ligand may also be an aptamer. The term "aptamer" is known in the state of the art. Preferably the term aptamer relates to a short nucleic acid or peptide molecule capable of specifically binding to a target molecule. Nucleic acid aptamers essentially consist of strands of RNA or DNA or derivatives thereof with a length of 15 to 100, more particularly of 20 to 60 nucleotides. Peptide aptamers are either isolated peptides or protein scaffolds comprising loop of peptide residues designed to bind to a target.

Preferably, the target is a biological target. The term "biological target" relates to any kind of organic molecule, but preferentially to peptides and derivatives thereof, nucleic acids and derivatives thereof, carbohydrates and derivatives thereof. For example, the biological target may be chosen from the group of peptides, peptidomimetics, antibodies, nucleic acids, carbohydrates, saccharides, oligosaccharides, polysaccharides, and glycosylated peptides, as defined already above in the context of biological ligands.

The target may be a small molecule or an entity of higher molecular weight.

Preferably, the target is a naturally occurring molecule or essentially consists of a naturally occurring molecule or a suitable fragment thereof.

Preferably, the target is a peptide as defined above, most preferably a naturally occurring peptide or essentially consisting of a naturally occurring peptide or a suitable fragment thereof. The target may also be functionally defined as an antigen or a hapten.

The nature of the target will be largely defined by the interest of the investigator concerned. For example, if the investigator is interested in analyzing or finding a ligand directed to a certain protein, then he may choose this protein or a suitable fragment thereof as a target. For example, if the investigator is interested in analyzing or finding a ligand capable of inhibiting the activity of a particular enzyme, he may choose to use the catalytically active domain of the enzyme to be his target. In such case, any biological ligand identified as being capable of binding to the target would constitute a very good candidate for a steric inhibitor of the enzyme.

The candidate for a biological ligand can be an entity of known or unknown structure.

In many biological screening applications, the general structure of the candidate will be known, e.g. the class of molecule (e.g. whether it is a peptide, a nucleic acid, an antibody, or a carbohydrate) and the approximate size or molecular weight of the candidate (e.g. the length of a peptide candidate). However, the detailed structure of the candidate may not be known. For example, it may be the case in applications directed to finding or optimizing a biological ligand from a random library or from a pool of mutated biological ligands. Such methods are known and are employed e.g. in affinity selection in phage display or in combinatoric selection procedures such as SELEX (systematic evolution of ligands by exponential enrichment), which is being used e.g. in the development of nucleic acid aptamers.

However, in some situations even the detailed structure of the candidate may be known, for example if candidates are provided by means of a library of known biological molecules. In a more specific example, an investigator may suspect a certain class of proteins or enzymes to interact with his target or receptor molecule. He may then choose to provide a library of such known proteins and enzymes and to analyze by means of the present invention whether any one of the proteins or enzymes interacts with his target or receptor molecule of interest.

From the above considerations, it will be evident that the candidates can be provided as a library. The term "library" is known to the person skilled in the art. Preferably, the term "library" is intended to relate to a library of candidates for biological ligands as defined above. A library may allow to analyze a multitude of candidates in parallel, which has several advantages: It is more time-efficient than analyzing each candidate separately and it may allow to analyze a large number of candidates without any prejudice, hypothesis, or considerations beforehand about their capability of binding to the target. The number of candidates to be analyzed is only limited by logistical considerations, but a typical library may contain more than 10, preferably more than 30, more preferably more than 100, more preferably more than 300, more particularly more than 1000, most particularly more than 10000 different candidates. The number of candidates to be analyzed in parallel will be determined largely by practical considerations. For example, the analysis in the format of 24, 48, 96, 386, 1024 analyses (including appropriate controls) in parallel will be advantageous, as there are analyzers and pipetting systems available for such formats in many laboratories, particularly in microtiter plate format. However, there are also higher density systems available, on suitable membranes, microarrays, microchips or multi-well slides. In summary, the analysis of libraries, particularly so-called "high-throughput screening", is a tool of high importance in drug discovery, drug optimization, and in the elucidating of biochemical interaction or signal transduction pathways.

Therefore, the invention also relates to a method for screening of a biological library comprising at least 10, 100, 1000, or 10000 biological ligands for ligands which bind with higher or lower affinity than other ligands comprised in the library.

The methods provided herein are of particular use in analyzing or screening libraries and in high-throughput approaches. In analyzing libraries, particularly in high-throughput screening it will be much appreciated that the invention provide reliable and fast methods which can also help to save washing steps and/or expensive reagents.

In a preferred embodiment, the library is a bacteriophage library.

As already mentioned, candidates are preferably contacted with the target under the same conditions and/or in parallel. The term "same conditions" preferably means that the binding conditions should be the same, for example that the same binding buffer should be used. The term "in parallel" means that the contacting of the target should occur at the same time or within the same reaction, for example on the same microtiter plate, microarray, or membrane using the same reactants from a common pool. More preferably, the term "in parallel" means that the contacting should occur within the same solution, for example in the same well of a microtiter plate, e.g. using a mixture of candidates.

Step (1) or step (2) of the method may be preceded by binding of the target or the candidate for a ligand to a solid support. In the case of analysis or screening of biological libraries, it may be advantagous to immobilize the candidates for ligands on the support and to supply the target in the mobile phase to all candidates. Then, any target moleculas binding with low affinity or low specificity can be removed or eluted by means of the present invention and it is possible to analyze (particularly to detect, identify, and/or isolate) the candidate(s) to which the target has bound with high affinity and/or specificity. The target may be labeled by any suitable means in order to allow for easier identification of binding. However, bound target may also be detected by any inherent property of the target or the complex (e.g. light absorption of a particular frequency by the target or the complex). One attractive method to analyze or detect the ligand-target complex may be surface plasmon resonance.

The support may be any support deemed appropriate by the person skilled in the art, e.g. a microtiter plate, a microarray, a microchip, a glass slide (e.g. a micro-well slide), a membrane (e.g. a nylon membrane, as being used e.g. in Western Blotting), or a bead or microbead (e.g. a magnetic bead). Particularly, the support may be made from polystyrene (PS) and derivatives thereof, such as high impact polystyrene (HIPS, polybutadiene bonded to polystyrene), acrylonitrile butadiene styrene (ABS), or any polystyrene copolymers (e.g. copolymers with divinylbenzene). Polystyrene derivatives also include derivatives generated by post-molding surface modification, e.g. with oxygen-rich plasmas, in order to introduce polar groups. Such groups allow for easy immobilization of peptides. Supports made from polystyrene and derivatives thereof are e.g. available in microtiter plate format.

In a further embodiment, the method according to the invention may comprise the additional step (4) of analyzing whether the candidates remains bound to the target or the candidate remains not bound to the target. In the case of a multitude of candidates, the method may comprise the step (4) of detecting and/or identifying the candidates remaining bound to the target or the candidates not remaining bound to the target. A candidate remaining bound to the target may be considered a biological ligand according to the invention.

A biological ligand analyzed according to the invention preferably binds to the target non-covalently but with high affinity and/or specifically. The person skilled in the art is familiar with the concepts of "high affinity" and "specific binding" in this context as they have long been known, e.g. from the context of antibody binding or from the context of inhibiting or activating enzymes.

More particularly, the term "high affinity" should be understood as already defined elsewhere in this specification.

"Specific binding" according to the present invention preferably means that the ligand should not bind substantially to ("cross-react" with) another target, more particularly to another target of similar composition but different structure, e.g. to a related (or "homologous") protein or peptide. Preferably, the specifically bound target should be bound with at least 3 times higher, more preferably at least 10 times higher and even more preferably at least 50 times higher affinity than any other relevant target. Which target may be used for comparison in such context will largely depend on the purpose for which the ligand is to be used. For example, if the ligand is later to be used as a means for detecting or staining a target protein or carbohydrate epitope in a sample, e.g. on a histological section, then the term specific binding means that the ligand should not substantially bind to (or cross-react with) other structures present in such sample or section. If the ligand is to be used as an inhibitor or activator of a given target (e.g. an enzyme), the ligand should preferably inhibit the target enzyme with at least 3 times higher, more preferably at least 10 times higher and even more preferably at least 50 times higher strength than any other relevant target enzyme present in the sample.

In the majority of cases, "high affinity" binding of the ligand to the target will at the same time mean that the ligand binds to the target "specifically".

However, it is also possible to choose binding conditions which will directly favor specific over unspecific binding. For example, it is possible to add a suitable competitor during the step of bringing into contact of the candidates and the ligand or during a subsequent wash step. Such competitor may be a molecule of similar structure as the target (e.g. a peptide considered homologous to a target peptide). Preferably, such competitor will differ from the target only in the epitope or region of interest.

Although the methods according to the invention have the advantage of allowing to detect or identify specifically binding biological ligands, it should be noted that non-specific binding ligands may be tolerable in many applications, for example if the target of interest can still be detected unequivocally, e.g. by separating the target of interest according to its size (e.g. by electrophoresis), or by the target's relatively higher abundance in the sample compared to unspecifically bound targets.

Preferably, the ultrasound is applied to the target-bound candidate(s) in a liquid or solution having acidic pH. It has been found in the context of the invention that acidic pH further improves the stringency or the selectivity and sensitivity of the present method (see Examples). The term "acidic pH" is known and relates to a pH value lower than pH 7. More preferably, the pH should be 6 or lower, more preferably 5 or lower, more preferably 4 or lower, more preferably 3.5 or lower, more preferably 3 or lower, most preferably 2.8 or lower. Preferably, but depending on the acid stability of the ligand and the target, the pH should not be lower than 1.5, more preferably not be lower than 1.8, most preferably not be lower than 2. The acid stability of a ligand or target can be easily estimated by the person skilled in the art. The upper and lower limits given above can be combined as considered appropriate by the person skilled in the art. A preferred pH range may therefore be between pH 1.5 and pH 7, more preferably between pH 2 and pH 5, more preferably between pH 2 and pH 3.5, most preferably between pH 2 and pH 3. In a screening or selection method, the pH may be lowered in each round of selection or in each washing step, e.g. from pH 5 to pH4 to pH 3 (see e.g. D'Mello, F. & Howard, C.R. J. Immunol. Method. 247, 191-203 (2001)).

The ultrasound can be applied to the target-bound candidate(s) at any temperature deemed suitable by the person skilled in the art. The choice of the temperature may e.g. depend on the temperature stability of the candidate(s) for a ligand or the target and can be chosen accordingly. The temperature stability of a candidate ligand or target can easily be estimated by the person skilled in the art. The temperature may also depend on the purpose of a biological ligand to be analyzed, e.g. a temperature of approximately 37 ºC may be suitable in cases of ligands which are eventually to be used as drugs in the human body. Preferably, the temperature should be 1 ºC or higher, more preferably 2 ºC or higher, more preferably 5 ºC or higher, more preferably 10 ºC or higher. Preferably, the temperature should not be higher than 95 ºC, more preferably not higher than 90 ºC, more preferably not higher than 80 ºC, more preferably not higher than 60 ºC, more preferably not higher than 50 ºC. Said upper and lower limits can be combined as considered appropriate, e.g. a suitable temperature range could from 1 ºC to 95 ºC, or 10 ºC to 50ºC. In most cases, the ultrasound application may be carried out at room temperature (15 ºC to 25ºC, more preferably 18 ºC to 25ºC).

The ultrasound application may be combined with any other methods or washing or elution steps and conditions considered appropriate by the person skilled in the art. For example, it may be possible to combine the ultrasound application with the presence of a specific or non-specific competitor in the washing liquid. For example, if the target is a particular nucleic acid, it may be considered to add an unspecific competitor such as sonicated salmon sperm DNA in order to reduce unspecific binding of a candidate to the ligand.

### Figure Legends

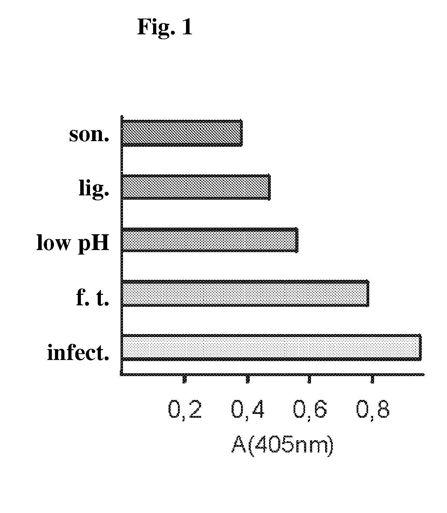

Fig. 1: Comparison of elution strategies relative efficacy. Isolated high-affinity clone GTFAHPQ was incubated over the immobilized streptavidin, washed with PBST and eluted with the respective method (sonication (i.e. application of ultrasound); ligand competition; low pH; free target; elution by infection). In this example, free target means that free streptavidin was added to the solution (which acts as a displacer of phage bound to immobilized streptavidin). In this example, free ligand means that biotin was added to the solution (biotin is a higher affinity ligand of streptavidin and acts to displace the peptide ligand GTFAHPQ). The amount of uneluted phage was detected by ELISA (see Supplementary Methods). son., sonication; lig., ligand; low pH; f.t., free target; infect., elution by infection.

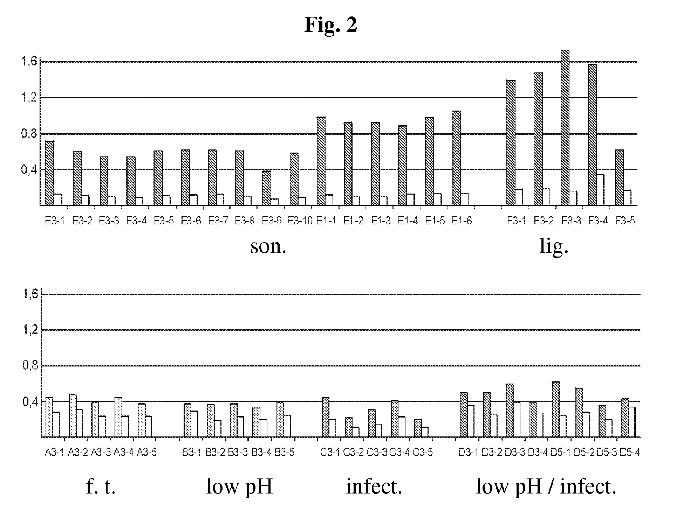

Fig. 2: Results of the ELISA assay of individual clones: Individual phage clones obtained with different elution strategies (Table 1) were tested for their affinity to bind streptavidin. Elution by sonication and elution with ligand (biotin) produced phages with the highest affinity toward streptavidin. All sequenced phages carried the same cyclic heptapeptide GTFAHPQ. All other elution strategies failed to select HPQ motif. In selection protocol D, phage clones D5-1 and D5-2 contained QPPGPSY motif. In comparison to GTFAHPQ, affinity determined in ELISA was smaller while affinity for blocked surfaces was suspiciously high. son., sonication; lig., ligand; f.t., free target; infect., elution by infection.

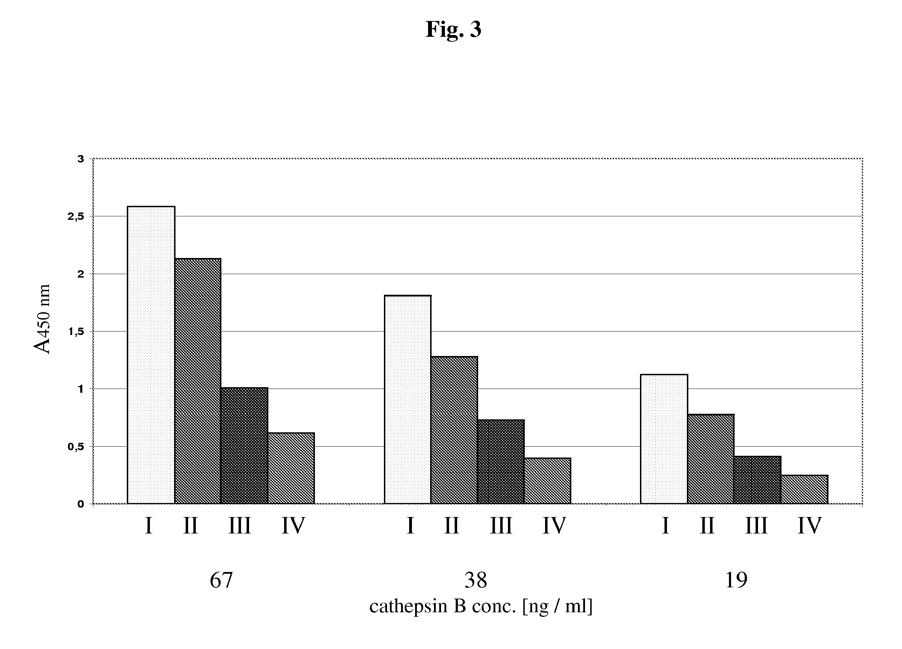

Fig. 3: Detection of residual cathepsin B in MTP wells after different elution conditions. The elution conditions were as follows: (I) control (no elution performed), (II) PBS pH 7,4 + sonication, (III) Gly-HCI pH 2,2, (IV) Gly-HCI pH 2,2 + sonication. The numbers below the table indicate the concentration of cathepsin B during binding to the rat polyclonal antibody [ng/ml] (67, 38, and 19 ng/ml]. The height of the bars reflects the absorption measured in the ELISA and thus serves as a relative value of residual cathepsin B.

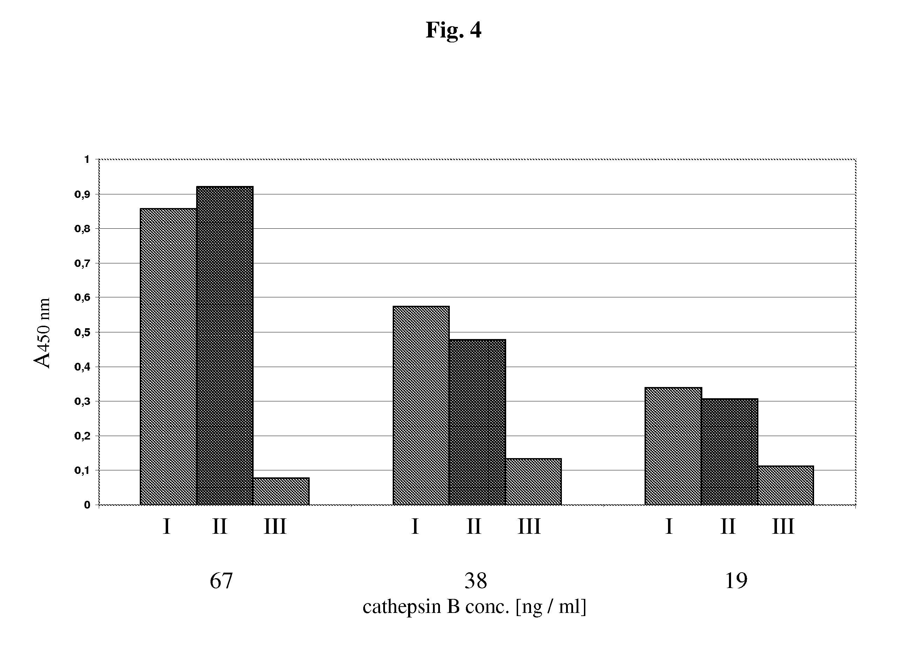

Fig. 4: Detection of cathepsin B in different eluates. The elution conditions were as follows: (I) Gly-HCl pH 2,2 + sonication, (II) Gly-HCl pH 2,2, (III) PBS pH 7,4 + sonication. The numbers below the table indicate the concentration of cathepsin B during binding to the rat polyclonal antibody [ng/ml] (67, 38, and 19 ng/ml] in experiment A. The height of the bars reflects the absorption measured in the ELISA and thus serves as a relative value of cathepsin B.

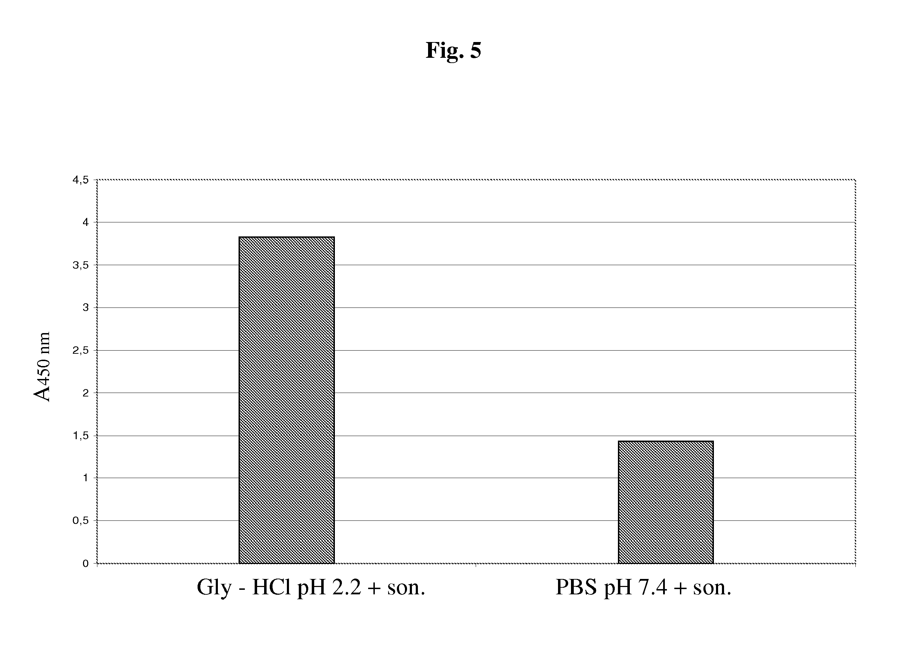

Fig. 5: Detection of rabbit anti-cathepsin B polyclonal antibody eluted samples (average values). son., sonication.

The references cited in this specification and the Examples have been searched and cited solely to provide for an easier understanding of the invention. No citation should be construed as an admission that the cited reference constitutes common general knowledge in the field.

The following Examples serve to illustrate the invention and should not be construed to limit the scope of the invention in any way.

### Example 1

Streptavidin was used as the target for affinity selection of peptides from a low-avidity type 3 phage display library (i.e. a maximum of 5 copies of peptides are displayed per virion as fusions to coat protein pIII). Biotin was used for competitive elution due to extremely strong interaction with streptavidin (K_{d} ~ 10⁻¹⁵ M, see Green, N.M. Adv Protein Chem 29, 85-143 (1975)) and well-characterized peptide biotin mimotopes previously reported and reviewed (Smith, G.P & Petrenko, V.A. Chem Rev. 97, 391-410 (1997); Lowman, H.B. Annu. Rev. Biophys. Biomol. Struct. 26, 401-24 (1997); Katz, B.A. & Cass, R.T. J. Biol. Chem. 272, 13220-8 (1997)). A number of nonselective elution strategies (Table 1, protocols A-D) were compared in selection efficiency to biotin elution (Table 1, protocol F).
All protocols based on nonselective elution of bound phage failed to yield peptides with HPQ tripeptide motif, a biotin mimotope in binding streptavidin, see also protocols involving free streptavidin elution (Table 1, protocol A) and elution as a result of direct bacterial infection (Table 1, protocol C). Surprisingly, protocols involving elution with Gly-HCI buffer (Table 1, protocols B and D) were also incapable to select for HPQ tripeptide motif, even with repetitive elution steps. This could neither be attributed to too stringent washing procedure, as competitive elution with biotin (Table 1, protocol F) under similar condition was effective nor could it be attributed to unfinished selection, since HPQ bearing clones were no longer present in the enriched libraries after the third selection round and sequencing resulted in single non-related peptide motif (see Methods).
Here, an improved nonselective elution method is presented (Table 1, protocol E; Methods). In this example, ultrasound elution at low pH is being used, preceded by rigorous washing. This technique achieved efficient selection of desired HPQ-tripeptide motif displaying clones in as little as one selection round. It has to be noted though, that in case a larger diversity of peptide ligands is preferred, washing stringency should be lowered.
Finally, the efficiency of all employed elution strategies concerning streptavidin-phage clone GTFAHPQ (Fig. 1) interaction was quantitatively compared. Ultrasound elution proved to be the most effective, followed by competitive elution by biotin, low pH elution, free target and direct bacterial infection.
In the present experiments, all commonly used elution strategies previously reported have failed to elute and select high affinity HPQ bearing clones. The disruption of phage-target interaction by sonication enabled single step selection of high affinity clones.

**Table 1: Overview of selection protocols. Brief description of washing and elution steps is given. For detailed description of protocols see Methods. The outcome was estimated as positive when the signal in ELISA affinity assay of selected clones exceeded blank by at least four-fold (see Fig. 2).**

| PROTOCOL | A | B | C | D | E | | F |
|---|---|---|---|---|---|---|---|
| ELUTION STRATEGY | free target | low pH | elution by infection | low pH/ elution by infection | sonication | | ligand |
| WASHING STEP | 10 x PBST | 30 to 35 x PBST | 10 to 35 x PBST | 10 to 35 x PBST | 25 x PBST | | 25 x PBST |
| | | | 4 to 16 x Gly-HCl | | 4 x Gly-HCl | | 4 x Gly-HCl |
| ELUTION STEP | streptavidin 100 µg/ml 1h | 8 to 10 x Gly- HCl | bacterial host (OD₆₀₀0.1)1h | 4 to 10 x gly-HCl | Gly-HCl 10 min sonication | | 0.1 mM biotin 60 min |
| | | | | bacterial host (OD₆₀₀ 0.1) 1h | | | |
| No. OF SELECTION ROUNDS | 3 | 3 | 3 | 1 to 5 | 1 | 3 | 3 |
| OUTCOME | - | - | - | - | + | + | + |

### Methods

Lyophilized streptavidin was obtained from Sigma-Aldrich, Steinheim, Germany. Maxisorp surface 96-well microtiter plates were purchased from Nalge Nunc International, Roskilde, Denmark. Random cyclic heptapeptide phage display library Ph.D.-C7C was procured from New England Biolabs, Beverly, MA, USA. HRP/Anti-M13 Monoclonal Conjugate was obtained from Amersham Biosciences, Little Chalfont, UK. All chemicals used were of analytical grade. In streptavidin selections, pre-coated and pre-blocked microtiter plates from Boehringer Mannheim, Mannheim, Germany were used.

Pre-coated and pre-blocked microtiter plates were incubated with an aliquot of 2x10¹¹ phages diluted to 100 µl with PBST (phosphate buffered saline containing 0,1% Tween 20) buffer (135 mM NaCl, 3 mM KCl, 10 mM Na₂HPO₄, 2 mM KH₂PO₄; pH 7.4) for 1 h at room temperature with gentle agitation. Selection of peptides from phage library was carried out according to the manufacturer's instructions with some modification. Non-binding phages were discarded by washing the wells with different washing strategies. Bound clones were eluted with different elution strategies.
For all sonication experiments, a water bath (Brand: ISKRA pio d.o.o.; Model: SONIS 3 GT) was used, at a frequency of 50 kHz and a power of 300 W).

### Washing and Elution Step in Protocols A-F (Table 1):

In **protocol A**, all three selection rounds were performed in a same manner. Unbound phage clones were washed with 0.2% PBST ten times and eluted with 100 µl 100 µg/ml of streptavidin at room temperature for one hour with gentile agitation.

In **protocol B**, thirty-fold PBST washings in the first round and thirty-five-fold PBST washings in the second selection round were applied. For each PBST washing, 200 µl of 0.2% PBST buffer was transferred into the well and shaken for five minutes at 50 rpm and then discarded. Eight consecutive eluates in the first round and ten in the second round were separately combined and amplified. For each glycine-HCl eluate, 100 µl of 0.2 M glycine-HCl buffer pH 2.2 was transferred into the microtiter well and shaken for five minutes at 100 rpm collected from the microtiter well and neutralized with 100 µl of 200 mM phosphate buffer pH 7.5. The same amount of this buffer was also used to neutralize microtiter well between glycine-HCl elutions and then added to the eluates.

In **protocol C**, the first round of selection was performed with 10 PBST, followed by 4 glycine-HCl washings. The second round was performed with 20 PBST and 8 glycine-HCl washing and the third round was performed with 35 PBST and 16 glycine-HCl washing. All these were discarded and the remaining phages were eluted by direct bacterial infection. 100 µl of *E. coli* ER2738 host cells (OD₆₀₀ ~ 0.1) were added to the well and shaken at 50 rpm for 1 hour. Next, infected host cells were transferred to fresh LB medium and further amplified.

**Protocol D** included five rounds of selection. In each round, we gradually increased the number of washings with PBST from 10 to 35-fold. After discarding non-binding phages, elution step followed. In each selection round, the number of glycine-HCI eluates, obtained as described in protocol B, was also increased from 4 to 10. Subsequently, remaining phages were also eluted by direct bacterial infection. All eluates were combined and amplified.

Elution by sonication (**protocol E**) included washing 25-fold with PBST and four-fold with glycine-HCl. Next, microtiter plate well was filled with 100 µl 0.2 M Gly-HCl pH 2.2 and emerged in sonificated water bath (50 kHz) for 10 minutes. Eluate was transferred in to 100 µl of neutralization buffer (200 mM phosphate buffer pH 7.5). The same amount of this buffer was used to neutralize microtiter well after glycine-HCl/sonication elution and then added to the eluate. Three such rounds of selection were performed.

**Protocol F** included three selection rounds. Washing steps were as in protocol E, followed by elution with 0.1 mM biotin solution for 1 hour at 50 rpm.

### Presence of High Affinity Clones in Enriched Libraries Obtained in Protocols A, B and C:

After the third round of selection of protocols A, B and C, two further rounds of selection were carried out. After ten-fold washing with 0.2% PBST (as in protocol A), phages were eluted with 0.1 mM biotin solution for 1 hour at 100 rpm.

Eluates were amplified by infecting *E. coli* ER2738 host cells. After 5 hours growth at 37 ºC, bacteria were removed by centrifugation and phages in the supernatant were precipitated by adding 1/6 volume of PEG/NaCl solution (20% polyethylene glycol-8000, 2.5 M NaCl) and overnight incubation at 4 ºC. The precipitate was resuspended in a small volume of PBS and amplified eluates were titered to determine phage concentration.
Finally, eluates from the last round of selection were used to infect plated bacterial host cells and 10 plaques were randomly selected. Individual phage clones were then grown and purified for further analysis.

### Phage ELISA:

Microtiter plate wells (Maxisorp, Nalge Nunc International) were coated with 100 µl of streptavidin solution (100 µg/ml) in 50 mM NaHCO₃ pH 8.5 overnight and blocked with 200 µl blocking buffer (2% bovine serum albumin in PBS) for 1 hour. A separate set of wells was blocked with blocking buffer without previous streptavidin immobilization as negative controls. 100 µl of each selected amplified phage clone were diluted to 200 µl with blocking buffer and transferred to coated wells. Plates were incubated for 1.5 hours at room temperature. Wells were then washed 3 times with 0.1% PBST. Horseradish peroxidase-labeled mouse anti-M13 monoclonal antibody (Amersham Biosciences, Little Chalfont, UK) in blocking buffer (1:5000), 200 µl per well, was added and incubated for 1 hour at room temperature. Finally, wells were washed 4 times with 0.1% PBST. 200 µl of substrate solution (0.22 mg/ml ABTS in 50 mM citric acid, 1.7 µl 30% H₂O₂/ml; pH 4.0) was added and incubated for 30 min at 37°C. Absorbance at 405 nm was then determined using a microtiter plate reader.

### DNA Sequencing

Single-stranded DNA from amplified selected phage clones was isolated by denaturing coat proteins with iodide buffer (10 mM Tris-HCl (pH 8.0), 1 mM EDTA, 4 M Nal) and precipitation with ethanol. Purified DNA was sequenced by MWG Biotech sequencing service (Munich, Germany).

### Efficacy of Elution Strategies:

Pre-coated and pre-blocked microtiter plates were incubated with an aliquot of 4x10¹⁰ pfu of clone GTFAHPQ diluted to 100 µl with 0.1% PBST for 1 h at room temperature with gentle agitation. Next, wells were washed ten-fold with 0.2% PBST and eluted using different elution strategies as described in protocols A, B, C, E and F. Wells were washed once again with 0.1% PBST and incubated with anti-M13 monoclonal antibody. Subsequently, ELISA test was performed as previously described.

### Example 2

For all sonication experiments, a water bath (Brand: ISKRA pio d.o.o.; Model: SONIS 3 GT) was used, at a frequency of 50 kHz and a power of 300 W).

### Experiment A:

Rabbit polyclonal antibody (pAb) against cathepsin B was adsorbed to MTP (microtiter plate), to which cathepsin B was added. Cathepsin B (and/or cathepsin B and pAb separately and/or cathepsin B-pAb complex) was eluted with:
a. low-ph buffer,
b. low-ph buffer + sonication, or
c. neutral-ph buffer + sonication.
All the eluates were saved for later analysis.

Cathepsin B was subsequently detected in an ELISA assay with secondary Ab, conjugated to horse-radish peroxidase.

Wells filled with low-ph buffer and sonicated gave the lowest signals, indicating most stringent elution, while wells filed with neutral-ph buffer and sonicated gave higher signals, indicating least effective elution (Fig. 3).

### Experiment B:

Eluates from previous experiment were neutralized (if appropriate) and eluted proteins allowed to adsorb to a new set of MTP wells. Both, cathepsin B (Fig. 4) *and* pAb against cathepsin B (Fig. 5) could be specifically detected in eluted samples with horse radish peroxidase conjugated anti-cathepsin B Ab or anti-rabbit Ab, respectively.
Signals of sonicated low-pH treated samples were highest while those eluted with ultrasound at neutral pH were lowest (Fig. 4 and 5). This indicates that breaking of protein-protein and/or protein target-MTP interactions by ultrasound in combination with low-ph buffer is even more effective than by sonication at neutral pH. Thereby, it is shown that the use of ultrasound during elution, particularly in low-ph buffer, is clearly beneficial to phage display affinity selection (demonstrated also by successful selection to streptavidin), as it contributes also to target protein desorption (see also below), enabling efficient detachment of phages which would remain bound to target despite of lowered pH to low pH elution conditions (i.e. the best binders).

However, ultrasound may also serve to detach the target molecule from the MTP surface. Binding of phage-displayed peptides to target protein has to be considered a dynamic equilibrium. If there are multiple copies of displayed peptides present on the phage capside (indeed, in our case there are up to 5) that means that phage remains bound to its target even though most (all but one) candidate ligands (here: peptides) are actually free at a given time point. Removing the target from the MTP surface by sonication results in elution of phage in solution. Once in solution, (1) the target concentration is significantly lower compared to solid surface and (2) phage can still infect the bacterial hosts even if most peptides are complexed to target protein.

## Claims

1. A method for analyzing the binding of a biological ligand to a target, comprising the steps of
(1) providing one, two, or more different candidates for a biological ligand,
(2) contacting said candidate(s) with a target, wherein for each candidate the target is the same or has the same structure,
(3) applying ultrasound to the target-bound candidates for a ligand.

2. The method according to claim 1, wherein the method comprises the additional step of
(4) detecting the candidate(s) remaining bound to the target or the candidate(s) not remaining bound to the target.

3. The method according to claims 1 or 2, wherein the ultrasound is applied to the target-bound candidate(s) in a liquid having acidic pH.

4. The method according to claim 3, wherein the pH is 3 or lower.

5. The method according to any of claims 1 to 4, wherein the ultrasound is applied during a wash step.

6. The method according to any of claims 1 to 5, wherein the method comprises the additional step of (4) isolating the candidate(s) remaining bound to the target or the candidate(s) not remaining bound to the target.

7. The method according to any of claims 1 to 4, wherein the ultrasound is applied during an elution step.

8. The method according to any of claims 1 to 7, wherein the candidate for a biological ligand is chosen from the group of peptides, peptidomimetics, antibodies, nucleic acids, carbohydrates, saccharides, oligosaccharides, polysaccharides, and glycosylated peptides.

9. The method according to any of claims 1 to 8, wherein the target is a biological target, preferably chosen from the group of peptides, peptidomimetics, antibodies, nucleic acids, carbohydrates, saccharides, oligosaccharides, polysaccharides, and glycosylated peptides.

10. The method according to any of claims 1 to 9, wherein the method is for screening of a biological library comprising at least 10, 100, 1000, or 10000 biological ligands for ligands which bind with higher or lower affinity than other ligands comprised in the library.

11. The method according to claim 10, wherein the biological library is a bacteriophage library.

12. The method according to any of claims 1 to 11, wherein the power of ultrasound is 50 to 400 W.

13. The method according to any of claims 1 to 12, wherein the frequency of ultrasound is 15 to 60 kHz.

14. A method of screening a biological library comprising the step of applying ultrasound to elute biological ligands which are specifically bound to a given target.

15. Use of ultrasound for eluting a specifically bound ligand from a target.
